(19) Europäisches Patentamt / European Patent Office / Office européen des brevets

(11) **EP 2 251 435 B1**

(12) **EUROPEAN PATENT SPECIFICATION**

(45) Date of publication and mention
of the grant of the patent:
**16.10.2013 Bulletin 2013/42**

(51) Int Cl.:
***C12Q 1/68*** *(2006.01)*     ***G01N 27/414*** *(2006.01)*

(21) Application number: **10174104.9**

(22) Date of filing: **02.03.2007**

(54) **Sensing apparatus for monitoring nucleic acid amplification, using an ion-sensitive field effect transistor (ISFET) for pH sensing**

Messgerät zur Überwachung einer Nukleinsäure-Amplifikation, das einen ionensensitiven Feldeffekttransistor (ISFET) zur pH-Bestimmung verwendet

Appareil de mesure pour suivre l'amplification des acides nucléiques, utilisant un transistor à effet de champ sensible aux ions (ISFET) pour determiner le pH.

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**17.11.2010 Bulletin 2010/46**

(62) Document number(s) of the earlier application(s) in accordance with Art. 76 EPC:
**07726604.7 / 2 129 792**

(73) Proprietor: **DNA Electronics Ltd**
**London WC1N 2EB (GB)**

(72) Inventors:
- **Toumazou, Christofer**
  **London, SW7 2AZ (GB)**
- **Ou, Chung-Pei**
  **London, SW4 7QH (GB)**

(74) Representative: **Icely, Dominic Michael et al**
**Marks & Clerk LLP**
**Fletcher House**
**Heatley Road**
**The Oxford Science Park**
**Oxford OX4 4GE (GB)**

(56) References cited:
WO-A1-02/086162    WO-A1-2005/075638
WO-A2-03/054225    WO-A2-03/073088
US-A1- 2005 136 423    US-A1- 2006 016 699
US-A1- 2006 088 839

- **PURUSHOTHAMAN S ET AL: "Protons and single nucleotide polymorphism detection: A simple use for the Ion Sensitive Field Effect Transistor" SENSORS AND ACTUATORS B, ELSEVIER SEQUOIA S.A., LAUSANNE, CH LNKD- DOI: 10.1016/J.SNB.2005.06.069, vol. 114, no. 2, 26 April 2006 (2006-04-26), pages 964-968, XP005349398 ISSN: 0925-4005 [retrieved on 2006-04-26]**
- **SAKURAI T ET AL: "REAL-TIME MONITORING OF DNA POLYMERASE REACTIONS BY A MICRO ISFET PH SENSOR" ANALYTICAL CHEMISTRY, AMERICAN CHEMICAL SOCIETY, US LNKD- DOI: 10.1021/AC00041A040, vol. 64, no. 17, 1 January 1992 (1992-01-01), XP009015113 ISSN: 0003-2700**

**Description**

**Field Of The Invention**

The present invention relates to quantitative real time monitoring of nucleic acid amplification, e.g. polymerase chain reaction (PCR) amplification, ligase chain reaction or transcription mediated amplification, by employing a pH sensitive ion-sensitive field effect transistor (ISFET) to detect proton release arising from primer extension as amplification proceeds. The invention provides sensing apparatus including a pH sensitive ISFET for this purpose.

**Background to the invention**

Quantitative real time polymerase chain reaction (qPCR or RT-PCR) has become a de facto standard for amplification of low amounts of DNA or RNA, e.g. for cloning of gene sequences, forensic testing and genetic testing for disease-linked mutations. Most embodiments of qPCR require labelled probes (e.g. fluorescent dyes) to detect amplicons. qPCR disclosed herein avoids the need for labelled probe. It relies instead on pH sensitive ISFET sensing of proton release consequent to PCR cycling and can thereby be performed on a chip.

As reported in published International Patent Application WO 03/073088, the inventors previously found that ISFETs can be used to monitor proton release associated with individual nucleotide insertion at the end of an oligonucleotide chain. Monitoring of individual nucleotide insertions by a pH sensitive ISFET may be utilised in DNA sequencing based on conventional Sanger method DNA sequencing and in identifying allelic variants, e.g. single nucleotide polymorphisms (SNPs), relying on detecting extension of oligonucleotide primers designed to target specific nucleic acid sites. The present invention arises from the further realisation by the inventors that protons are also a PCR product and that qPCR may therefore also be achieved by ISFET monitoring of proton release, preferably in a low reaction volume chamber.

WO2005/075638 discloses the use of a GenFET with an immobilised probe to detect a target gene, which if present results in a charged nucleic acid abutting the surface. The whole GENFET array is exposed to a volume containing a sample containing the gene.

**Summary of the invention**

Thus, disclosed is a method of monitoring nucleic acid amplification in a sample comprising a buffered nucleic acid amplification mixture for amplification of target sequence if present in the sample, characterised in that said monitoring is by means of detecting change of pH resulting from proton release in the presence of target sequence as amplification proceeds beyond a threshold number of cycles for buffering capacity of the sample to be overcome, said detecting employing a sensing apparatus comprising an ISFET having a sensing surface exposed to the sample and arranged to generate an electrical output signal in response to change of pH at said transistor surface and means for detecting an electrical output signal from the ISFET. To achieve the required degree of sensitivity in detection, amplification will preferably be carried out in small (preferably nano) volumes and at low buffer capacity such that the number of protons released leads to rapid change in pH as the buffer capacity of the sample is overcome. Thus such a method may advantageously be carried out in a nanoreactor with integrated pH sensitive ISFET provided in a microfluidic device or chip.

In a first aspect, the present invention provides a sensing apparatus for monitoring nucleic acid amplification in a sample comprising a silicon substrate integrating one or more heating elements arranged to heat a sample and more than one ISFET-containing reaction chambers each with an ISFETs to measure the pH of said sample.

Preferably, a sensing surface of the one or more ISFETs is provided with a silicon nitride layer. The sensing apparatus preferably further comprises one or more temperature sensors and, in a preferred option, the temperature sensors may be arranged to control the one or more heating elements so as to control the temperature of the sample.

The reaction chambers preferably receive the sample in or on a base, or the apparatus further comprises channels to receive the sample in or on a base. Reaction chambers of 1 pl to about 10 $\mu$l contain said sample and overly the one or more ISFETs.

The sample may be caused to flow through a channel or chamber of a microfluidic device and as it flows is subjected consecutively to different temperatures whereby thermo-cycling for PCR is achieved. The sample may be caused to flow through a chamber or channel which passes consecutively through different temperature zones suitable for said thermocycling. The sample may be is caused to flow through a channel which passes consecutively through different temperature zones provided in the base of said microfluidic device, said zones being suitable for successive repeats along the channel of the PCR stages of denaturing, primer annealing and primer extension.

More than one ISFET is preferably employed for sensing of pH change of the sample at different positions along said channel. The sample may be moved backwards and forwards in a microchamber between required temperature zones for thermocycling and said ISFET is provided in a wall of said chamber. Preferably, at least one of: a reference electrode, a reference FET having a non-enzyme linked layer, and a conductivity sensor; are further integrated with the silicon substrate.

The sensing apparatus may further comprises reagents for amplification of DNA. The reagents may be DNA, optionally including probes to capture target DNA in the sample, immobilised on beads. The reagents may also be dried and located within a reaction chamber or arranged to be introduced to the reaction chamber with the sample.

WO 03/054225 discloses (see Figure 3 page 15-16) a system where hybridization takes place in a recessed zone created by etching layer 7. The remaining volume allows the fluid to mix freely about. The prior art thus disclose at most one chamber, that being the enclosed volume above the array of sensors.

## Brief Description Of The Drawings

Specific embodiments of the invention will now be described by way of example only with reference to the accompanying figures, in which:

Figure 1 shows pH changes occurring during DNA chain extension using a buffered reaction medium.

Figure 2 is a schematic diagram of a field effect transistor as previously utilised for DNA sequencing.

Figure 3 is a schematic diagram of a pair of field effect transistors as previously employed for DNA sequencing.

Figure 4 is a schematic representation of results obtained using the pair of field effect transistors for DNA sequence determination of the Sanger type on a DNA template employing all required dNTPS and a single ddNTP in the reaction mixture.

Figure 5 shows diagrammatically PCR cycling resulting in amplification of a target nucleic acid sequence.

Figure 6 shows proton release through DNA extension monitored in applying the invention to DNA sequence determination.

Figure 7 shows results of simulated quantitative real time PCR using ISFET pH sensing.

Figure 8 is a schematic diagram of a microfluidic device containing an embedded ISFET in a low volume reaction chamber for carrying out qPCR.

Figure 9 is a further schematic view of a microfluidic device for carrying out PCR monitoring with a stationary sample in a reaction chamber/well (19) with cover (20). The chip may be heated by the heating element (21) and cooled to each required temperature in sequence for thermocycling of the sample.

Figure 10 shows a plan view and cross-section of a microfluidic device for carrying out PCR monitoring in accordance with the invention wherein the sample flows through a microfluidic channel (22), e.g. in a platform of acrylic or Perspex, which consecutively crosses zones of a silicon chip base (23) maintained at different temperatures for DNA denaturation, primer hybridisation to target and primer extension respectively. The three temperature zones for thermo-cycling of the sample for PCR are shown.

Figure 11 is a schematic view of an alternative microfluidic device for monitoring PCR in accordance with the invention in which the sample is moved backwards and forwards in a chamber between different temperature zones to achieve the required thermo-cycling.

## Detailed description

### pH sensitive ISFETs

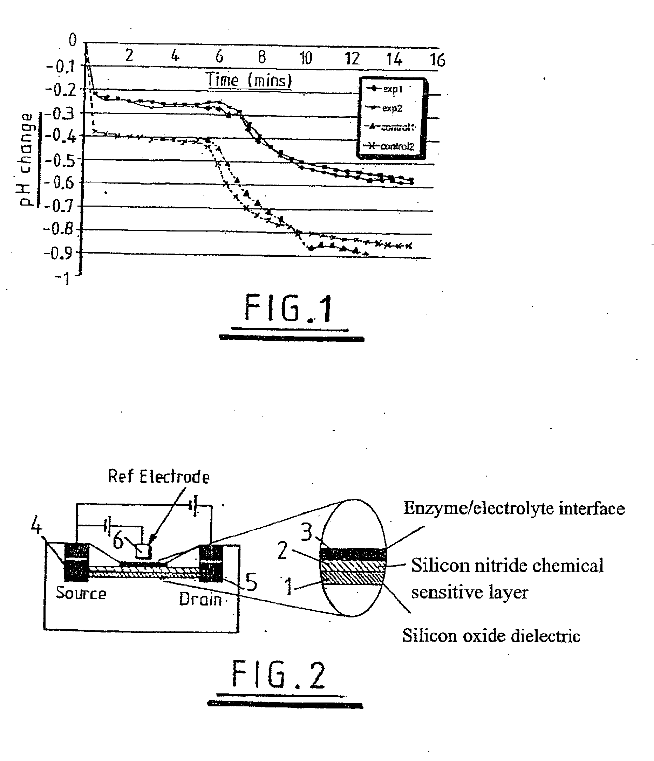

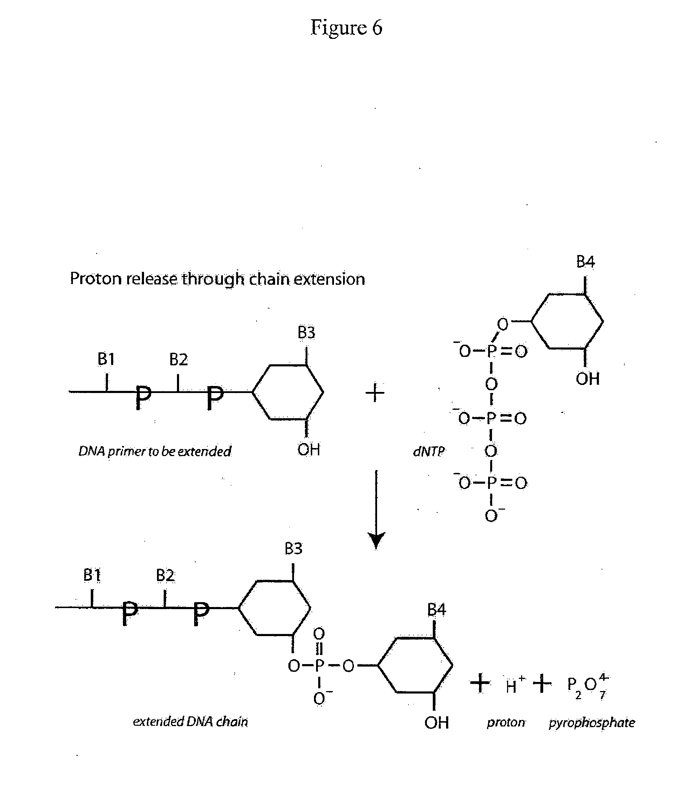

As indicated above, WO 03/073088 teaches use of pH sensitive ISFETs for DNA sequencing of the Sanger-type relying on the fact that incorporation of nucleotide bases to a growing DNA chain leads to release of hydrogen ions (see Figure 6). The results shown in Figure 1 demonstrate the DNA extension reaction and its effect on pH. The pH was measured using a glass electrode arrangement, with measurements of the absolute value of pH taken every 30 seconds. ISFETs capable of detecting individual nucleotide insertions at the end of a growing nucleotide chain during such DNA extension are now equally proposed for monitoring nucleic acid amplification. As previously described, such an ISFET may, for example, be provided with an ion sensitive silicon nitride layer on top of which is a layer of polymerase. The ISFET is able to detect rapid pH changes and has an immediate response rate measured to be within 1 ms of a pH change [Woias et al., 'Modelling the short-time response of ISFET sensors', Sensors and Actuators B, 24-25 (1995), 211-217]. The follow-up reactions which succeed nucleotide insertion are pH-dependent, and therefore net consumption or production of hydrogen ions depends on the pH in which the reaction occurs. Generally, the reaction is conducted in the pH range 6 to 8.5, e.g. 7 to 7.5. In this range, hydrogen ions are overall liberated during nucleotide insertion.

A pH sensitive ISFET as may be used for monitoring DNA extension or nucleic acid amplification is shown in Figure 2. As described in WO 03/073088, this ISFET comprises a silicon oxide dielectric layer 1, a silicon nitride chemical sensitive layer 2, and an enzyme/electrolyte interface 3. The layers 1, 2 and interface 3 are located between a source 4 and drain 5 (the conventional configuration of a FET). The FET is provided on a silicon chip, which is encapsulated in epoxy resin to protect it from the reagent mixture. The epoxy resin helps to protect the FET from hydration and charge migration [Matsuo and Esashi, 'Methods of ISFET fabrication', Sensors and Actuators, 1 (1981) 77-96]. The FET gate itself is not covered by epoxy resin so that it may be immersed in the reagent mixture.

The enzyme/electrolyte interface 3 shown in Figure 2 allows ion sensitivity of the silicon nitride layer 2 to be used for DNA sequencing or detection of protons for monitoring of nucleic acid amplification. The FET functions by producing an exchange of charged ions between the surface of the chemical sensitive layer 2 and the reacting medium (i.e. the enzyme/electrolyte interface 3):

$$SiOH \xleftrightarrow{E} SiO^- + H^+$$

$$SiOH_2^+ \longleftrightarrow SiOH + H^+$$

$$SiNH_3^+ \longleftrightarrow SiNH_2 + H^+$$

The inclusion of silicon nitride is advantageous because it provides increased and faster sensitivity to changes of pH than would be obtained in the absence of the silicon nitride. In addition the silicon nitride helps to protect the FET from hydration and charge migration.

A non-Nernstian response accounts for the immediate sensitivity of the FET, arising from rapid proton dependant binding and unbinding of charged ions at the insulating gate silicon nitride surface, which results in a reproducible variation in the voltage drop across the silicon nitride layer 2. The variation of the voltage drop across the silicon nitride layer 2 correlates with changes of pH. The voltage drop is monitored using instrumentation circuitry, thereby allowing the detection of individual nucleotide insertions. The measured voltage is referred to as the flatband voltage.

The enzyme/electrolyte interface 3 is deposited on the silicon nitride layer using a known enzyme linkage method [Starodub et al., 'Optimisation methods of enzyme intergration with transducers for analysis of irreversible inhibitors', Sensors and Actuators B 58 (1999) 420-426]. The method comprises pre-silanising the silicon nitride layer 2 using aminosilane solution, and then activating the surface using glutaraldehyde. A drop of buffer/polymerase enzyme solution is then deposited on the silicon nitride layer 2 and allowed to dry for about half an hour to form the enzyme layer 3.

The embodiment shown in Figure 2 uses a reference electrode 6 to provide a measurement of pH changes. The reference electrode is relatively large and difficult to fabricate. An alternative embodiment does not use a reference electrode, but instead uses a second FET which has the same construction as the first FET, but is provided with a non-enzyme linked layer instead of the enzyme layer 3. This configuration is advantageous because it provides a differential measurement which gives an improved signal to noise ratio.

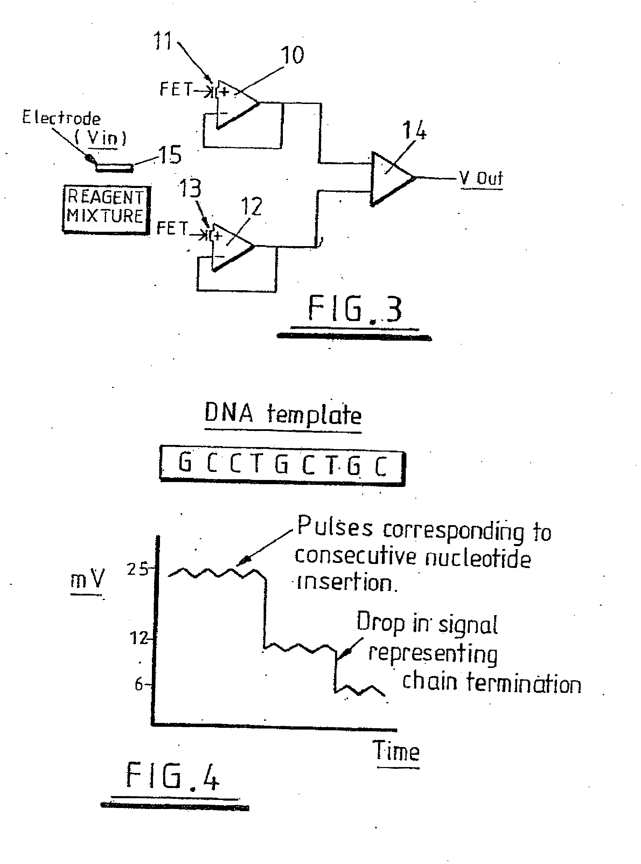

A further alternative embodiment is illustrated in Figure 3 and has also previously been described in WO 03/073088 but solely in the context of monitoring of DNA extension. The configuration of this embodiment is based upon a known construction [Wong and White, 'A self- Contained CMOS Integrated pH Sensor, Electron Devices meeting IEEE 1988] which has previously been used to monitor gradual slow drift of pH. The embodiment comprises a first operational amplifier 10 to which the source of the first FET 11 is connected (the first FET has the enzyme linked layer), and a second operational amplifier 12 to which the source of the second FET 13 is connected (the second FET has no-enzyme linked to the FET). The drains of the first and second FETs are connected to a fixed current source (not shown). Outputs from the first and second operational amplifiers are passed to a differential amplifier 14, which amplifies the difference between the outputs to generate an output signal $V_{out}$. Negative feedback from the differential amplifier 14 passes to a noble metal electrode 15 which is located in the reagent mixture. The operational amplifier 14 generates an output voltage which keeps the voltage applied to the FETs 11, 13 the same despite changes of hydrogen concentration. The embodiment shown in Figure 3 is advantageous because it allows rationalisation of fabrication of the FETs 11, 13 and the operational amplifiers 10, 12, 15.

The FETs 11, 13 may be arranged to form the first stage of the operational amplifiers 10,12. This is done for each operational amplifier by replacing a conventional FET of a long tail pair located at the input of the operational amplifier, with the first or second FET 11, 13. This is advantageous because it allows the first and second FETs to form part of the amplification circuitry.

A schematic example of a flatband voltage detected using the embodiment shown in Figure 3 for DNA sequencing is illustrated in Figure 4. The flatband voltage consists of pulses representing pH changes associated with nucleotide insertion and drops corresponding to ddNTP insertion and chain termination. The number of local pulses prior to a larger drop determines the number of bases present before termination at a known base; the magnitude of the larger drop is dependant on the ratio of ddNTP: dNTP used in the reagent mixture and is important due to the dependence of read length for that drop. Through repetition of the process four times in different reaction chambers containing each of the four ddNTPS separately, the complete sequence is delineated.

The steps used to fabricate the ion sensitive FET are set out below:

PURIFIED SILICON SUBSTRATE
ADDITION OF DOPANT: PRODUCTION OF p-TYPE SUBSTRATE
SURFACE OXIDATION: $SiO_2$ LAYER GENERATION
SOURCE/DRAIN DEFINITION AND IMPLANTATION
SILICON NITRIDE DEPOSITION USING LPCVD*
CONTACT FORMATION
PASSIVATION

*Low pressure Chemical Vapour Deposition

The FETs and in particular those shown in Figure 3, and the amplification stages may be replaced or combined with PMOS transistors.

*Monitoring of nucleic acid amplification*

A pH sensitive FET in a sensing device format as shown in Figure 2 or 3 may also be advantageously employed to monitor nucleic acid amplification in accordance with uses of the present apparatus. Such use of a pH sensitive FET will be further described below with reference to performing qPCR. However, it will be appreciated that a pH sensitive FET may be employed in the same manner to monitor any form of nucleic acid amplification including transcription mediated amplification (TMA) or ligase chain reaction (LCR).

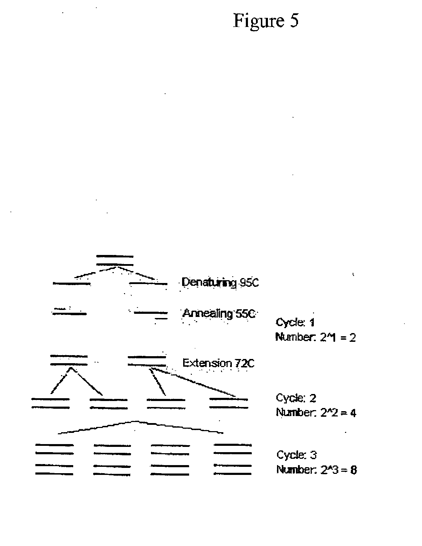

PCR is a process of amplification of a fragment of double-stranded DNA wherein primers are provided for hybridisation to each DNA strand to target a specific sequence of interest for amplification over a number of cycles of the amplification process. There are three stages to a PCR cycle as shown in Figure 5. These are effected through thermal cycling in the presence of the necessary components for primer extension including a heat- resistant DNA polymerase as follows:

- denaturing: the double-stranded template DNA is separated into two single strands due to the breaking of the hydrogen bonds that connect the two DNA strands, e.g. at 95 °C;
- annealing: the sequence of interest is defined by two oligonucleotide primers which hybridise to the single strands of template DNA, carried out at e.g. 55 °C;
- extension: DNA polymerase in the presence of dNTPs extends each primer, e.g. at 72° C

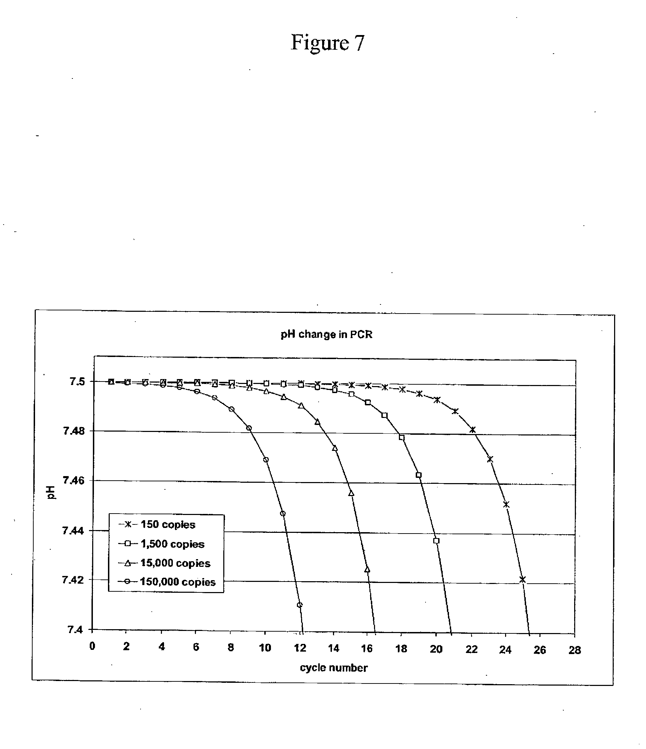

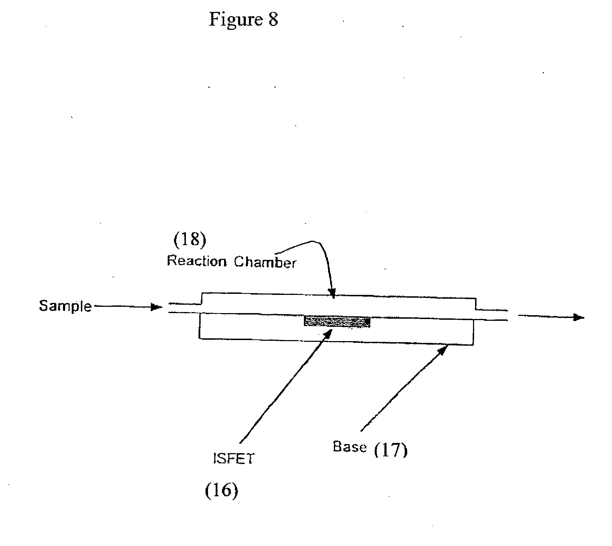

In the extension stage of the cycle, protons are released consequent to base addition to each primer. It is this proton release which is used as the basis for real time quantification of PCR product in accordance with uses of the present apparatus. As noted above, the ISFET sensing surface will be exposed to buffered amplification mixture, preferably in a low volume reaction chamber or channel, e.g. a chamber or channel of a microfluidics device. Thus, for example, advantageously a low volume reaction chamber or well may be employed, e.g. of volume 1pl to about 10μl. This may overlay an ISFET as shown in Figure 8 in which the ISFET (16) is embedded in the base (17) of the reaction chamber or well (18) and microchannels are provided for sample delivery and exit from the chamber. The chamber is capable of being heated and cooled between the annealing and denaturing temperatures (e.g. between 55 and 95° C) for PCR thermocycling. Reagents for amplification such as the primers and dNTPs may be either dried inside the reaction chamber or introduced with the sample. If the target sequence is present, then this can be determined by the pH drop exceeding a certain threshold as the PCR cycles progress. If the target sequence is not present, the primers will not anneal to the template DNA and proton-releasing primer extension will not occur so that there is no significant change of pH.. Due to the presence of buffer in the amplification mixture, pH change resulting from PCR cycling will initially be countered by buffer action. However, once the buffering capacity is overcome, rapid change in pH will be measureable as shown in Figure 7. The number of cycles before the pH change passes a given threshold will depend on the DNA template concentration, the higher the concentration the fewer the cycles thereby allowing quantification of template DNA by calibrating the number of PCR cycles to reach the threshold against known template loads.

A number of ISFET-containing chambers as described above may be provided in a single microfluidic chip. This may be of particular benefit for example where amplification of short tandem repeats (STRs) is desired for DNA fingerprinting or where there is desire to amplify multiple DNA samples from the same source or different sources for genetic testing, or amplify different sites of one DNA strand. The ISFET, housed in a microfluidic chamber, may be an integral part of a chip such as a silicon chip with resistive on-chip heating elements and temperature sensors to control the temperature for DNA hybridisation and extension. Such a chip may also provide an integrated reference electrode and conductivity sensors.

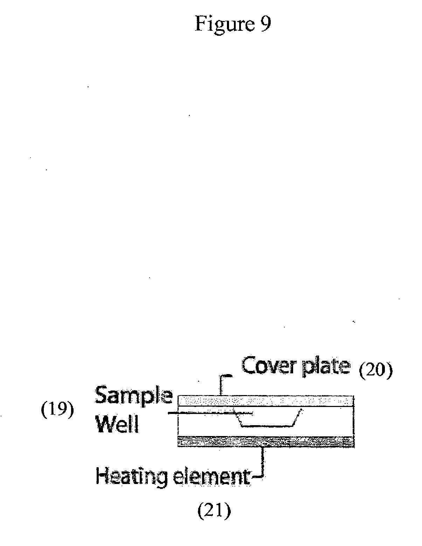

Fabrication of one or an array of nanoliter reactor chambers in silicon with integrated actuators (heaters) for PCR monitoring has been described for example in Iordanov et al. 'Sensorised nanoliter reactor chamber for DNA multiplication, IEEE (2004) 229-232. Chambers thus fabricated (see Figure 9) might each be provided with an integrated ISFET for monitoring of nucleic acid amplification in accordance with uses of the present apparatus. As noted by Iordanov et al. in their above-noted paper, untreated silicon and standard silicon-related materials are inhibitors of Taq polymerase. Therefore, when silicon or a silicon-related matererial, e.g. silicon germanium or strained silicon (all such materials will hereinafter be referred to as a silicon substrate) is employed for fabrication of a microchip chamber or channel for nucleic acid amplification it will usually be covered with material to prevent reduction of polymerase efficiency by the silicon such as SU8, polymethyl-methacrylate (PMMA), Perspex™ or glass.

Surface passivation of microfabricated silicon-glass chips for PCR is also described by Shoffner et al. in Nucleic Acid Res. (1996) 24, 375-379. In their studies, silicon chips were fabricated using standard photolithographic procedures and etched to a depth of 115 $\mu$m. Pyrex™ glass covers were placed on top of each silicon chip and the silicon and glass were anodically bonded. Several types of surface passivations were investigated with a view to improving PCR amplification efficency with thermo-cycling in the provided chamber. An oxidised silicon surface ($SiO_2$) was found to give consistent amplifications comparable with reactions performed in a conventional PCR tube. Such a surface may also be favoured in fabricating a micofluidic device for carrying out nucleic acid amplification with ISFET pH sensing. For further discussion of surface passivation in the fabrication of PCR microfluidic devices reference may be made to Zhang et al. 'PCR microfluidic devices for DNA amplification' in Biotechnology Advances (2006) 24, 243-284. As described in that review article, as an alternative to static surface passivation by substrate coating, it may be possible to include a passivation agent in the sample (dynamic passivation).

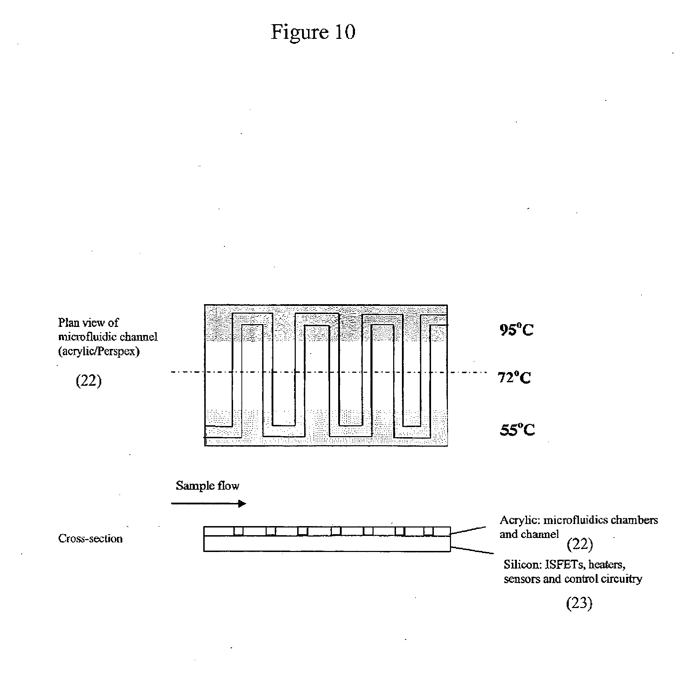

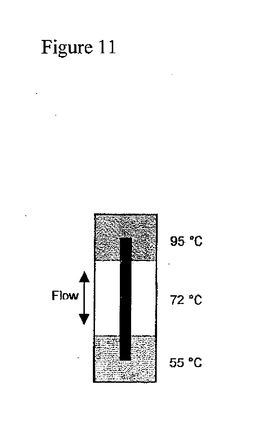

As an alternative to low reaction volume chambers as described above for carrying out PCR monitoring in a stationary sample, the sample for PCR monitoring may be caused to flow through a channel or chamber of a microfluidic device and as it flows is subjected consecutively to different temperatures whereby thermo-cycling for PCR is achieved. Thus, for example, the sample may be caused to flow through a channel or chamber which passes consecutively through different temperature zones suitable for the PCR stages of denaturing, primer annealing and primer extension, e.g. a channel in a microfluidic device, e.g. a silicon chip device, which passes consecutively through zones of different temperature provided in the base suitable for successive repeats along the channel of the PCR stages of denaturing, primer annealing and primer extension. Such microfluidic structures for performing continuous flow nucleic acid amplification on a chip are described, for example, by Auroux et al. in 'Minaturised nucleic acid analysis' Lab Chip (2004) 4, 534-546 and may be combined with ISFET monitoring of amplification. Microfluidic structures of this type as illustrated in Figures 10 and 11 may be fabricated through the use of standard microfabrication techniques using for example photolithography to define the fluidic network and then an etching or deposition step to create the required channel or channels, for example in a PMMA, acrylic, Perspex™ or glass substrate. A cover plate in glass or PMMA or other material may or may not be overlaid to cover the channels. The base of the channel(s) may be formed by substrate bonding to a silicon chip with integrated ISFET and temperature sensors as well as heating or heat pump (Peltier) elements, such that the reaction mixture is in direct contact with these sensors and actuators, and may or may not include circuitry for temperature control. Alternatively, the base of the channel(s) may be formed by a printed circuit board (PCB) housing ISFET and temperature sensors such that these are in direct contact with the reaction mixture. The surface of the PCB will need to be levelled such that it provides a planar base onto which the microfluidic platform can be overlaid. The PCB may also house heating or heat pump elements, sensor interface and temperature control circuitry. Reagents present within the microfluidic channel or chamber will be those of the buffered amplification mixture, ncluding the primers chosen for ability to hybridise to the target at sites suitable for amplification of the chosen sequence, the required enzyme(s) for amplification and all four dNTPs in excess.

Thus, in one embodiment for monitoring of PCR with thermo- cycling, the sample for nucleic acid amplification is caused to flow through a microfluidic channel (22) (e.g. microfabricated in a platform of acrylic, PMMA or Perspex) on a substrate (23), e.g. a silicon substrate of a silicon chip, and as it flows consecutively passes through temperature zones provided in the substrate or base suitable for successive repeats along the length of the channel of the stages of denaturing, annealing and chain extension, e.g. 95 °C for denaturation, 55 ° C for primer hybridisation to target and 72 ° C for primer extension with subsequent repetition (see Figure 10) . In this way, continuous flow- through amplification is achieved and may be monitored by means of one or more ISFETs embedded in the base. It may be preferred to employ ISFET detectors at a number of positions along the channel of a device as described above for continuous flow PCR such that real time PCR analysis can be achieved as amplification proceeds.

On- chip heating elements, such as polysilicon resistors may be used to heat the reaction mixture, using the Joule heating effect of power dissipated in a resistor when current is passed through it ($P=I^2R$) . Cooling could be achieved through heat dissipation through the silicon substrate and microfluidic platform, which is possible because of the small volumes of reaction mixture involved as well as the efficient thermal dissipation of the silicon chip base. Alternatively, an on- chip Peltier heat pump element for both heating and cooling could be implemented in known manner. Temperature uniformity is very important to avoid on- chip thermal gradients, both for the sake of the reaction mixture and for the sake of the ISFET and other on- chip circuitry. This may be achieved by an appropriate arrangement of the heating elements.

Temperature control could be achieved by a proportional- integral- derivative (PID) controller, which is one of the most common closed- loop feedback control systems. Errors between the measured temperature and the target temperature are used to calculate the level of heating required. Calculation of this output level is performed based on the current error directly (proportional), the history of the error (integral), and the predicted future error based on its rate of change (derivative) . Similarly, a PI controller would stabilise temperature based on present and historical values of the error as described in Iordanov et al. (2004) *ibid.* Alternatively, techniques such as pulse- width modulation or duty-cycling could be implemented. In these techniques, heater output is not adjusted by amplitude but by time for which the heater is "on" as a percentage of a fixed time period. The "on" time is inversely proportional to the error between the measured temperature and the target temperature, so as the target temperature is approached, the "on" time of the heating element is also reduced.

It may alternatively be chosen to have a reciprocating system (see Figure 11) whereby the amplification mixture is moved backwards and forwards in a microchamber between the required temperature zones for thermo-cycling. It will be appreciated that nucleic acid amplification resulting from such on chip sample-shunting PCR (described in the above-noted review article of Auroux et al.) may be monitored by providing an ISFET in a wall of the microfluidic chamber.

For further details of microfluidic devices for PCR, which may be modified for ISFET sensing in accordance with the invention, reference may again be made to Zhang et al. (2006) Biotech. Adv. 24, 243-284. As discussed in that review article, while such devices may preferably take the form of silicon chips, other materials for the chip substrate may be employed such as glass, various polymers and ceramics. As an alternative to contact heating for thermo-cycling, various non-contact heating methods may be employed as also discussed in the same review article, including by way of example hot-air mediated heating, utilisation of IR light, laser-mediated heating, induction heating and microwave irradiation

While the above described PCR systems are designed to achieve thermo-cycling, various isothermic nucleic acid amplification techniques are known, e.g. single strand displacement amplification (SSDA), and DNA or RNA amplification using such techniques may equally be monitored by ISFET detection in accordance with uses of the present apparatus.

Apparatus

It will be appreciated that in an aspect of the invention, there is provided a sensing apparatus for monitoring nucleic acid amplification in a sample, wherein said apparatus comprises a chamber or channel to receive the sample in or on a base, e.g. a silicon substrate, and one or more pH sensitive ISFETs are arranged for monitoring of nucleic acid amplification in said chamber or channel, said base as presented in said chamber or channel having a coating to improve amplification efficiency, i.e. said base has been subjected to surface passivation as discussed above. The chamber or channel will generally be provided in a microfluidic device. The microfluidic device may take any form as discussed above. Need for surface passivation of the base may however be avoided by choice of base material or if dynamic passivation is employed as already noted above.

Thus, the invention provides more generally a sensing apparatus for monitoring nucleic acid amplification in a sample wherein said apparatus comprises a microfluidic device in which a chamber or channel to receive the sample is provided in or on a base, e.g. a silicon substrate, and one or more pH sensitive ISFETs are arranged for monitoring of nucleic acid amplification in said chamber or channel. A low reaction volume chamber or well as discussed above may be provided for monitoring PCR in a stationary sample or a chamber or channel for continuous flow PCR. Further chambers or channels may be provided for simultaneous monitoring of multiple samples. As discussed above, the base may include heating elements and temperature sensors for thermocycling of the sample.

*Monitoring of DNA extension on beads*

Whether a pH sensitive ISFET is being used for DNA sequencing or nucleic acid amplification, e.g. qPCR, as an alternative to DNA extension being on DNA immobilized at the ISFET surface, DNA extension may occur on beads. Use of beads may be advantageously combined with use of an ISFET lying in a horizontal plane at the bottom of the reaction chamber or channel, e.g. as shown in Figure 8, such that the beads settle in the vicinity of the ISFET sensing surface. The beads may be chosen such that gravitational settlement alone brings the beads into the vicinity of the ISFET sensing surface. Alternatively, magnetic/metallic beads may be employed and magnetically drawn into the vicinity of the ISFET sensing surface. The beads may be spherical particles synthesised from any suitable material for the attachment of DNA, e.g. silica, polystyrene, agarose or dextran. The size of the beads may be adjusted to assist gravitational settling and to accord with need to avoid blockage of the reaction chamber and entry and exit ports. The beads can be washed off the sensor surface using water or buffer solution. Linkage of DNA to the beads may be achieved using conventional methods, e.g. functionalisation of the bead surface. A spacer may be employed. The coverage of the bead is controlled by adjusting the DNA to bead ratio. Where a pH sensitive ISFET is being used for DNA sequencing or monitoring of nucleic acid amplification and there is less size constraint, then, for example silica beads (e.g. about 200 nm diameter) may be preferably employed and DNA directly immobilized on the beads or immobilized following modification of the beads to provide a carboxylic functional group. Plastic beads (e.g. plastic microbeads of about 1 $\mu$m) may for example alternatively conveniently be employed. Where the ISFET is being employed to monitor nucleic acid amplification, the beads may carry probe DNA which captures target DNA in the sample.

*Use of DNA probe immobilised on the ISFET*

As an alternative to the use of beads, DNA probe for capture of target DNA may be linked directly or indirectly to the ISFET. Such immobilisation of DNA probe may be achieved using techniques well known for DNA probe immobilisation on a solid surface, e.g. such techniques well known from the DNA microarray art. Thus, DNA probe immobilisation on the ISFET may be achieved by in situ-oligonucleotide synthesis (by lithography or other means).

*Sample preparation for amplification*

Immobilisation of target probe as discussed above may be particularly favoured where the sample contains both target nucleic acid and unwanted nucleic acid. Immobilisation of the probe will be such as to enable separation of target from any unwanted nucleic acid or interfering proteins. By use of immobilised probe to bring the target into close proximity with the ISFET sensing surface benefit may be achieved of increasing signal to noise ratio by localising the pH change caused by nucleic acid amplification.

DNA for amplification may come from a variety of sources such as a mouth swab, a blood sample or cultured cells. Sample preparation for monitoring of nucleic acid amplification in accordance with the invention may include one or both of the steps of concentrating cells and release of the nucleic acid required for amplification from the cells, e.g. a cloned DNA sequence. These steps may be carried out separate from the device for nucleic acid amplification or integrated into part of the same device, e.g. a PCR chip as described above. Released nucleic acid for amplification may, for example, be further purified by binding to microparticles (beads) as described above. Thus extraction and purification of target DNA from a biological sample might be achieved on the same chip as PCR by employing an appropriate lab-on-the chip (LOC) method such as the laser-irradiated magnetic bead system (LIMBIS) as described in Lee et al., 'Microchip-based one step DNA extraction and real time PCR in one chamber for rapid pathogen identification', Lab Chip (2006) 6, 886-895.

*Kits*

Also disclosed are kits for detecting a target nucleic acid sequence in a sample by nucleic acid amplification, e.g. qPCR, which comprise a sensing device comprising a reaction chamber or channel and a pH sensitive ISFET for monitoring of nucleic amplification in said reaction chamber or channel as described above, e.g. such a sensing device in the form of a microfluidic chip, wherein primers for said amplification are provided in the reaction chamber or channel or separately within the kit. The primers may be provided together with bead-immobilized oligonucleotide probe for the target nucleic acid. As indicated above, the reaction chamber or channel of the sensing device may have other reagents dried within the reaction chamber including DNA polymerase and the required dNTPs for primer extension.

The following references provide additional background information relevant to the invention:

Shakhov and Nyrén, 'A Sensitive and Rapid Method for Determination of Pyophosphate Activity', Acta Chem. Scand. B 36 (1982) 689-694;

R. Buck, 'Electrochemistry of Ion-Selective Electrodes', Sensors and Actuators (1981) 1, 197-260;

Victorova et al, 'New substrates of DNA polymerases', FEBS Let. (1999) 453, 6-10; and

Hanazato et al., 'Integrated Multi-Biosensors Based on an Ion-sensitive Field-Effect Transistor Using Photolithographic Techniques', IEEE Transactions on Electron Devices (1989) 36, 1303- 1310.

The following example describes in more detail simulation of pH change with PCR cycles of DNA amplification and how this may be utilised for ISFET detection of target amplification.

**Example**

Figure 7 shows pH change with PCR cycles for a typical PCR amplification employing an amplification mixture of 10 $\mu$M Tris HCl, pH 7.5, 0.4 mM dNTPs, 2 mM MgCl$_2$, 50 mM KCl, 0.05% w/v BSA, 1 U per $\mu$l Taq polymerase, 1 $\mu$M PCR primer pair, and prepared DNA template (150- 150, 000 copies) . The hypothetical amplicon is 200 base- pairs and each dNTP incorporation releases one proton. The starting pH value is 7.5. Given the concentration of the Tris and dNTP, the buffer capacity at each pH value can be determined. The theoretical pH change can be determined by the number of protons released from the reaction and the buffer capacity at the pH where the reaction occurs.

As indicated above, the number of cycles before the pH change passes a given threshold will depend on the DNA template concentration, the higher the concentration the fewer the cycles thereby allowing quantification of template DNA by calibrating the number of PCR cycles to reach the threshold against known template loads.

***Calculation of theoretical pH change***

In a simplified version of the reaction, a single extension reaction can be described as below:

(1) $HP_3O_{10}^{-3}$- nucleoside + DNA- 3'OH => $H_2P_2O_7^{2-}$ +DNA- O- PO (O$^-$)- O- nucleoside
(2) $H_2P_2O_7^{2-}$ => $HP_2O_7^{3-}$ + H$^+$

The immediate element balance of the nucleotide insertion reaction is shown in (1), resulting in an extended DNA chain and pyrophosphate with a net charge of -2 (pKa$_1$ and pKa$_2$). In the pH range 6 to 8.5, some pyrophosphate will have the third proton (pKa$_3$) dissociated as shown in (2), resulting in a decrease in pH.

pKa of pyrophosphates:

pka$_1$ ~ 0.83
pKa$_2$ ~ 1.96
pKa$_3$ ~ 6.68
pKa$_4$ ~ 9.39

The calculation of pH change as the function of PCR cycle in a simplified version:

$$\text{Buffer capacity} \quad \beta = \frac{dn}{dpH} \quad ,$$

where *dn* is the increase of base and *dpH* is the change of pH value.

$$\text{Also } \beta \cong 2.303(\frac{[HA]_0 \, K_a [H^+]}{(K_a + [H^+])^2} ,$$

where $[HA]_0$ is the total concentration of the buffer chemical, such as TrisHCl, dNTP, DNA; Ka is the dissociation constant of the proton of the chemical; [H$^+$] is the proton concentration.

Given the initial pH value of the reaction 7.5, one can work out the total buffer capacity of the reaction mixture of 150 copies DNA, 10 $\mu$M TriHCl and 400 $\mu$M dNTP,

$$\beta = \beta_{Tris} + \beta_{dNTP} = 1.08 \times 10^{-4} M \text{ (DNA concentration is negligible)}$$

Then

$$pH_2 = pH_1 + \beta \times dn$$

$$= 7.5 + \frac{dn}{1.08 \times 10^{-4}}$$

Then a new buffer capacity can be calculated based on the new starting pH value and the anticipated pH change given the amount of proton produced.

**Claims**

1. A sensing apparatus for monitoring nucleic acid amplification in a sample, comprising: a silicon substrate integrating one or more heating elements arranged to heat a sample; and more than one ISFET-containing reaction chambers each with an ISFET to measure the pH of said sample.

2. A sensing apparatus as claimed in claim 1, wherein a sensing surface of the ISFETs is provided with a silicon nitride layer.

3. A sensing apparatus as claimed in claim 1 or claim 2, further comprising one or more temperature sensors.

4. A sensing apparatus as claimed in claim 3, wherein the temperature sensors are arranged to control the one or more heating elements so as to control the temperature of the sample.

5. A sensing apparatus as claimed in claim 1, wherein reaction chambers of 1 pl to about 10 $\mu$l contains said sample and overly the ISFETs.

6. A sensing apparatus as claimed in claim 1, wherein the sample is caused to flow through the reaction chambers of the microfluidic device and as it flows is subjected consecutively to different temperatures whereby thermo-cycling for PCR is achieved.

7. A sensing apparatus as claimed in claim 6 wherein said sample is caused to flow through the reaction chambers such that it passes consecutively through different temperature zones suitable for said thermocycling.

8. A sensing apparatus as claimed in claim 7 wherein said sample is moved backwards and forwards in the reaction chambers between required temperature zones for thermocycling and said ISFET is provided in a wall of each said chamber.

9. A sensing apparatus as claimed in any preceding claims further integrating with the silicon substrate at least one of: a reference electrode, a reference FET having a non-enzyme linked layer, and a conductivity sensor.

10. A sensing apparatus as claimed in any preceding claims further comprising reagents for amplification of DNA.

11. A sensing apparatus according to claim 10, wherein the reagents are DNA, optionally including probes to capture target DNA in the sample, and are immobilised on beads.

12. A sensing apparatus as claimed in anyone of claims 1 to 9, further comprising reagents for amplification of DNA, which reagents are dried and located within a reaction chamber or arranged to be introduced to the reaction chamber with the sample dried inside the reaction chamber or introduced with the sample.

**Patentansprüche**

1. Erfassungsvorrichtung zum Überwachen einer Nukleinsäureamplifikation in einer Probe, umfassend: ein Siliciumsubstrat, das ein oder mehrere Heizelemente enthält, die angeordnet sind, um eine Probe zu erhitzen; und mehr als eine ISFET-haltige Reaktionskammer, wobei jede mit einem ISFET versehen ist, um den pH-Wert der Probe zu messen.

**2.** Erfassungsvorrichtung nach Anspruch 1, wobei eine Erfassungsoberfläche der ISFET mit einer Siliciumnitridschicht versehen ist.

**3.** Erfassungsvorrichtung nach Anspruch 1 oder 2, ferner umfassend einen oder mehrere Temperatursensoren.

**4.** Erfassungsvorrichtung nach Anspruch 3, wobei die Temperatursensoren angeordnet sind, um das eine oder die mehreren Heizelemente zu steuern, um die Temperatur der Probe zu steuern.

**5.** Erfassungsvorrichtung nach Anspruch 1, wobei Reaktionskammern von 1 pl bis ungefähr 10 μl die Probe enthalten und über den ISFET liegen.

**6.** Erfassungsvorrichtung nach Anspruch 1, wobei bewirkt wird, dass die Probe durch die Reaktionskammern der mikrofluidischen Einrichtung fließt, und während sie fließt aufeinanderfolgend unterschiedlichen Temperaturen ausgesetzt wird, wodurch ein Thermocycling für eine PCR erzielt wird.

**7.** Erfassungsvorrichtung nach Anspruch 6, wobei bewirkt wird, dass die Probe durch die Reaktionskammern fließt, so dass sie aufeinanderfolgend unterschiedliche Temperaturzonen passiert, die sich für das Thermocycling eignen.

**8.** Erfassungsvorrichtung nach Anspruch 7, wobei die Probe in den Reaktionskammern zwischen erforderlichen Temperaturzonen für ein Thermocycling nach hinten und nach vorne bewegt wird und der ISFET in einer Wand jeder Kammer vorgesehen ist.

**9.** Erfassungsvorrichtung nach einem vorstehenden Anspruch, wobei das Siliciumsubstrat darüber hinaus zumindest eines des Folgenden beinhaltet: eine Referenzelektrode, einen Referenz-FET mit einer nicht-enzymverknüpften Schicht und einen Leitfähigkeitssensor.

**10.** Erfassungsvorrichtung nach einem vorstehenden Anspruch, ferner umfassend Reagenzien für eine Amplifikation von DNA.

**11.** Erfassungsvorrichtung nach Anspruch 10, wobei die Reagenzien DNA sind, optional enthaltend Sonden, um Ziel-DNA in der Probe zu fangen, und auf Beads immobilisiert sind.

**12.** Erfassungsvorrichtung nach einem der Ansprüche 1 bis 9, ferner umfassend Reagenzien für eine Amplifikation von DNA, wobei die Reagenzien getrocknet und innerhalb einer Reaktionskammer angeordnet sind oder angeordnet sind, um in die Reaktionskammer eingebracht zu werden, wobei die Probe innerhalb der Reaktionskammer getrocknet ist, oder um mit der Probe eingebracht zu werden.

**Revendications**

**1.** Appareil de détection pour surveiller l'amplification d'acide nucléique dans un échantillon, comprenant : un substrat de silicium intégrant un ou plusieurs éléments chauffants agencés pour chauffer un échantillon ; et plusieurs chambres de réaction contenant un ISFET chacune avec un ISFET pour mesurer le pH dudit échantillon.

**2.** Appareil de détection selon la revendication 1, dans lequel une surface de détection des ISFET est pourvue d'une couche de nitrure de silicium.

**3.** Appareil de détection selon la revendication 1 ou la revendication 2, comprenant en outre un ou plusieurs capteurs de température.

**4.** Appareil de détection selon la revendication 3, les capteurs de température étant configurés pour commander les un ou plusieurs éléments chauffants de manière à réguler la température de l'échantillon.

**5.** Appareil de détection selon la revendication 1, des chambres de réaction de 1 pl à environ 10 μl contenant ledit échantillon et surplombant les ISFET.

6. Appareil de détection selon la revendication 1, l'échantillon étant amené à s'écouler à travers les chambres de réaction du dispositif microfluidique et, au fur et à mesure qu'il s'écoule, étant soumis successivement à différentes températures de telle manière qu'un cyclage thermique pour la PCR soit obtenu.

7. Appareil de détection selon la revendication 6, ledit échantillon étant amené à s'écouler à travers les chambres de réaction de sorte qu'il traverse consécutivement différentes zones de température adaptées pour ledit cyclage thermique.

8. Appareil de détection selon la revendication 7, ledit échantillon étant déplacé vers l'arrière et vers l'avant dans les chambres de réaction entre les zones de température requises pour le cyclage thermique et ledit ISFET étant disposé dans une paroi de chacune desdites chambres.

9. Appareil de détection selon l'une quelconque des revendications précédentes intégrant en outre avec le substrat de silicium au moins l'un de : une électrode de référence, un FET de référence ayant une couche liée non-enzyme, et un capteur de conductivité.

10. Appareil de détection selon l'une quelconque des revendications précédentes comprenant en outre des réactifs pour l'amplification d'ADN.

11. Appareil de détection selon la revendication 10, les réactifs étant de l'ADN, comprenant facultativement des sondes pour capturer l'ADN cible dans l'échantillon, et étant immobilisés sur des billes.

12. Appareil de détection selon l'une quelconque des revendications 1 à 9, comprenant en outre des réactifs pour l'amplification d'ADN, lesdits réactifs étant séchés et situés dans une chambre de réaction ou configurés pour être introduits dans la chambre de réaction avec l'échantillon séché à l'intérieur de la chambre de réaction ou introduit avec l'échantillon.

FIG.1

FIG.2

11

10

FET→|(+

Electrode
( Vin )

15

14

REAGENT
MIXTURE

13

12

FET→|(+

V Out

FIG.3

DNA template

G C C T G C T G C

Pulses corresponding to
consecutive nucleotide
insertion.

mV    25

Drop in signal
representing
chain termination

12

6

Time

FIG.4

Figure 5

Denaturing 95C

Annealing 55C

Cycle: 1
Number: 2^1 = 2

Extension 72C

Cycle: 2
Number: 2^2 = 4

Cycle: 3
Number: 2^3 = 8

## Figure 6

Proton release through chain extension

# Figure 7

Figure 8

(18)
Reaction Chamber

Sample →

ISFET
(16)

Base (17)

Figure 9

# Figure 10

Plan view of
microfluidic channel
(acrylic/Perspex)

(22)

95°C

72°C

55°C

Sample flow

Cross-section

Acrylic: microfluidics chambers
and channel (22)

Silicon: ISFETs, heaters,
sensors and control circuitry

(23)

Figure 11

Flow

95 °C

72 °C

55 °C

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


**Patent documents cited in the description**

- WO 03073088 A
- WO 2005075638 A
- WO 03054225 A


**Non-patent literature cited in the description**

- **WOIAS et al.** Modelling the short-time response of ISFET sensors. *Sensors and Actuators B,* 1995, vol. 24-25, 211-217
- **MATSUO ; ESASHI.** Methods of ISFET fabrication. *Sensors and Actuators,* 1981, vol. 1, 77-96
- **STARODUB et al.** Optimisation methods of enzyme intergration with transducers for analysis of irreversible inhibitors. *Sensors and Actuators B,* 1999, vol. 58, 420-426
- **WONG ; WHITE.** A self- Contained CMOS Integrated pH Sensor. *Electron Devices meeting IEEE,* 1988
- **IORDANOV et al.** Sensorised nanoliter reactor chamber for DNA multiplication. *IEEE,* 2004, 229-232
- **SHOFFNER et al.** *Nucleic Acid Res.,* 1996, vol. 24, 375-379
- **ZHANG et al.** PCR microfluidic devices for DNA amplification. *Biotechnology Advances,* 2006, vol. 24, 243-284
- **AUROUX et al.** Minaturised nucleic acid analysis. *Lab Chip,* 2004, vol. 4, 534-546
- **ZHANG et al.** *Biotech. Adv.,* 2006, vol. 24, 243-284
- **LEE et al.** Microchip-based one step DNA extraction and real time PCR in one chamber for rapid pathogen identification. *Lab Chip,* 2006, vol. 6, 886-895
- **SHAKHOV ; NYRÉN.** A Sensitive and Rapid Method for Determination of Pyophosphate Activity. *Acta Chem. Scand. B,* 1982, vol. 36, 689-694
- **R. BUCK.** Electrochemistry of Ion-Selective Electrodes. *Sensors and Actuators,* 1981, vol. 1, 197-260
- **VICTOROVA et al.** New substrates of DNA polymerases. *FEBS Let.,* 1999, vol. 453, 6-10
- **HANAZATO et al.** Integrated Multi-Biosensors Based on an Ion-sensitive Field-Effect Transistor Using Photolithographic Techniques. *IEEE Transactions on Electron Devices,* 1989, vol. 36, 1303-1310